# EUROPÄISCHE PATENTSCHRIFT

(11) **EP 2 212 289 B1**
(45) Veröffentlichungstag und Bekanntmachung des Hinweises auf die Patenterteilung: **23.10.2013**
(21) Anmeldenummer: 08785831.2
(22) Anmeldetag: 05.09.2008
(51) Int. Cl.: C07D 213/61

(54) **VERFAHREN ZUR HERSTELLUNG VON 2,2-DIFLUORETHYLAMIN-DERIVATEN DURCH AMID-HYDRIERUNG**
METHOD FOR PRODUCING 2,2-DIFLUOROETHYLAMINE DERIVATIVES BY AMIDE HYDROGENATION
PROCÉDÉ DE PRÉPARATION DE DÉRIVÉS DE 2,2-DIFLUORÉTHYLAMINE PAR HYDRATATION D'AMIDES

(30) Priorität: 18.09.2007 EP 07116640
(43) Veröffentlichungstag der Anmeldung: 04.08.2010
(73) Patentinhaber: Bayer CropScience AG, 40789 Monheim (DE)
(72) Erfinder: LUI, Norbert, 51519 Odenthal (DE); ANTONS, Stefan, 51373 Leverkusen (DE)
(86) Internationale Anmeldenummer: PCT/EP2008/007271
(87) Internationale Veröffentlichungsnummer: WO 2009/036900

(56) Entgegenhaltungen:
- WO-A-2006/013939

## Beschreibung

Die vorliegende Erfindung betrifft ein Verfahren zur Herstellung von 2,2-Difluorethylamin-Derivaten ausgehend von 2,2-Difluoressigsäureamid-Derivaten. Weiterer Gegenstand der vorliegenden Erfindung sind die in diesem erfindungsgemäßen Verfahren als Ausgangsverbindungen eingesetzten 2,2-Difluoressigsäureamid-Derivate, deren Herstellung sowie deren Verwendung zur Herstellung von 2,2-Difluorethylamin-Derivaten.

Derivate von 2,2-Difluorethylaminen sind wichtige Zwischenprodukte zur Herstellung von agrochemischen Wirkstoffen. Entsprechende 2,2-Difluorethylamin-Derivate können beispielsweise in der Synthese von als insektizid wirksamen Enaminocarbonylverbindungen, beispielsweise von 4-Aminobut-2-enolidverbindungen, eingesetzt werden. Enaminocarbonylverbindungen, welche 2,2-Difluorethylaminobausteine umfassen, sind beispielsweise aus den internationalen Patentanmeldungen WO 2007/115644 und WO 2007/115646 bekannt.

Aus der WO 2007/115644 ist bekannt, dass man 2,2-Difluorethylamin-Derivate, wie beispielsweise die Verbindung der unten stehenden Formel (IIIa), durch Alkylierung des Amins der Formel (Ia) mit gegebenenfalls substituiertem Chlormethylpyridin der Formel (IIa) herstellen kann (Schema 1 der WO 2007/115644; vgl. Herstellung von Ausgangsverbindungen; Verbindungen der Formel (III); III-1: N-[(6-Chlorpyridin-3-yl)methyl]-2,2-difluorethyl-1-amin).

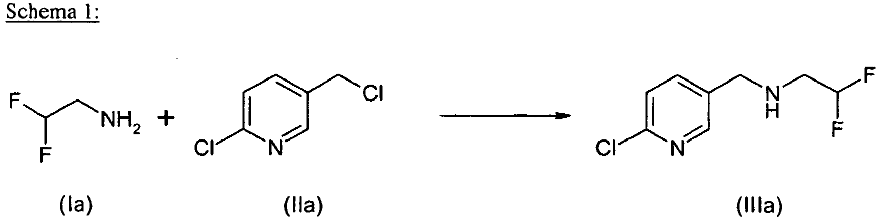

Nachteilig bei diesem Verfahren ist die geringe Ausbeute von 53 %, welche durch die mögliche Mehrfachalkylierung des Stickstoffatoms bedingt ist. Dieser Anteil der Mehrfachalkylierung kann nur durch den Einsatz eines großen Überschusses an Amin reduziert werden, was aber bei einem kostenintensiven Amin unwirtschaftlich ist.

Ausgehend von diesem Stand der Technik ergibt sich als Aufgabe der vorliegenden Erfindung, ein Verfahren zur Herstellung von 2,2-Difluorethylamin-Derivaten bereitzustellen, welches vorzugsweise einfach und kostengünstig durchzuführen ist. Die mit diesem angestrebten Verfahren erhältlichen 2,2-Difluorethylamin-Derivate sollen dabei vorzugsweise mit hoher Ausbeute und hoher Reinheit erhalten werden. Insbesondere soll das angestrebte Verfahren den Erhalt der gewünschten Zielverbindungen ohne die Notwendigkeit komplexer Aufreinigungsmethoden ermöglichen.

Gelöst wird diese Aufgabe durch ein Verfahren zür Herstellung von 2,2-Difluorethylamin-Derivaten.

Das erfindungsgemäße Verfahren ist dadurch gekennzeichnet, dass man gemäß Anspruch 1 2,2-Difluoressigsäureamid-Derivate der allgemeinen Formel (IV) zu den entsprechenden Zielverbindungen der allgemeinen Formel (III) gemäß nachfolgendem Schema 2 reduziert:

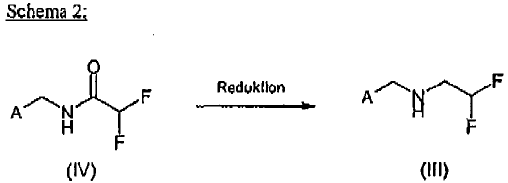

Erfindungsgemäß ist somit vorgesehen, dass die gewünschten 2,2-Difluorethylamin-Derivate der allgemeinen Formel (III) durch eine Reduktion der entsprechenden 2,2-Difluoressigsäureamid-Derivate der allgemeinen Formel (IV) hergestellt werden. Die gewünschten 2,2-Difluorethylämin-Derivate der allgemeinen Formel (III) werden unter den erfindungsgemäßen und weiter unten näher spezifizierten bevorzugten Reaktionsbedingungen mit guten Ausbeuten in hoher Reinheit erhalten, womit das erfindungsgemäße Verfahren die oben genannten Nachteile überwindet. Die gewünschten Verbindungen werden dabei in einer Reinheit erhalten, welche eine umfangreiche Aufarbeitung des unmittelbaren Reaktionsprodukts im Allgemeinen nicht erforderlich macht. Gegenüber dem aus dem Stand der Technik bekannten Verfahren, welches von einem zu alkylierenden Amin gemäß Schema 1 ausgeht, können die Ausbeuten durch das erfindungsgemäße Verfahren verbessert werden.

Im Rahmen der vorliegenden Erfindung wird unter einem Derivat ein von dem bezeichneten organischen Grundgerüst (Baustein) abgeleiteter Stoff ähnlicher Struktur bezeichnet, d.h. unter einem 2,2-Difluorethylamin-Derivat wird beispielsweise eine Verbindung verstanden, welche einen 2,2-Difluorethylamin-Baustein umfasst.

In den oben genannten allgemeinen Formeln (III) und (IV) weist der Rest A die folgende Bedeutung auf:
- Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder

- Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder
- 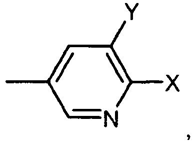 in welchem
   - X: für Halogen, Alkyl oder Halogenalkyl steht und
   - Y: für Halogen, Alkyl, Halogenalkyl, Halogenalkoxy, Azido oder Cyan steht.

Bevorzugte, besonders bevorzugte und ganz besonders bevorzugte Bedeutungen des in den oben erwähnten allgemeinen Formeln (III) und (IV) aufgeführten Restes A werden im Folgenden erläutert.
- A: wird bevorzugt ausgewählt aus der Gruppe, bestehend aus 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-1,3-thiazol-5-yl, 2-Chlor-pyrimidin-5-yl, 2-Trifluormethyl-pyrimidin-5-yl, 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3-yl, 5-Methyl-6-iod-pyrid-3-yl, 5-Difluormethyl-6-fluor-pyrid-3-yl, 5-Difluormethyl-6-chlor-pyrid-3-yl, 5-Difluormethyl-6-brom-pyrid-3-yl und 5-Difluormethyl-6-iod-pyrid-3-yl.
- A: wird besonders bevorzugt ausgewählt aus der Gruppe, bestehend aus 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl, 2-Chlor-pyrimidin-5-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl und 5-Difluormethyl-6-chlor-pyrid-3-yl.
- A: wird ganz besonders bevorzugt ausgewählt aus der Gruppe, bestehend aus 6-Chlor-pyrid-3-yl, 6-Brom-pyrid-3-yl, 6-Chlor-1,4-pyridazin-3-yl, 2-Chlor-1,3-thiazol-5-yl, 5-Fluor-6-chlor-pyrid-3-yl und 5-Fluor-6-brom-pyrid-3-yl.

Unter dem Begriff "Alkyl", entweder in Alleinstellung oder aber in Kombination mit weiteren Begriffen, wie beispielsweise Halogenalkyl, wird im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl, n-Heptyl, n-Octyl, n-Nonyl, n-Decyl, n-Undecyl und n-Dodecyl. Von diesen Alkylresten sind C₁-C₆-Alkylreste besonders bevorzugt. Insbesondere bevorzugt sind C₁-C₄-Alkylreste.

Unter dem Begriff "Aryl" wird erfindungsgemäß ein aromatischer Rest mit 6 bis 14 Kohlenstoffatomen, vorzugsweise Phenyl, verstanden.

Unter dem Begriff "Arylalkyl" wird eine Kombination von erfindungsgemäß definierten Resten "Aryl" und "Alkyl" verstanden, wobei der Rest im Allgemeinen über die Alkylgrupe gebunden wird; Beispiele hierfür sind Benzyl, Phenylethyl oder α-Methylbenzyl, wobei Benzyl besonders bevorzugt ist.

Im Rahmen der vorliegenden Erfindung werden durch Halogen substituierte Reste, beispielsweise Halogenalkyl, einfach oder mehrfach bis zur maximal möglichen Substituentenzahl halogenierte Reste verstanden. Bei mehrfacher Halogenierung können die Halogenatome gleich oder verschieden sein. Halogen steht dabei für Fluor, Chlor, Brom oder Iod, insbesondere für Fluor, Chlor oder Brom.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Gegebenenfalls substituierte Reste können einfach oder mehrfach substituiert sein, wobei bei einer Mehrfachsubstitutionen die Substituenten gleich oder verschieden sein können.

Die Reduktion der 2,2-Difluoressigsäureamid-Derivate der allgemeinen Formel (IV) zu den entsprechenden Aminen der allgemeinen Formel (III) kann mit dem Fachmann an sich bekannten Reduktionsmitteln erfolgen. Erfindungsgemäß ist es möglich, die Reduktion mit
- komplexen Hydriden,
- nichtkomplexenMetall-oderHalbmetallhydriden, d.h. in-situ erzeugten Borhydriden
- Hydriden von Si-Verbindungen der allgemeinen Formel

   H-Si-R₃,

   wobei R für H, gegebenenfalls substituiertes C₁-C₁₂-Alkyl, gegebenenfalls substituiertes Aryl oder Chlor steht, oder
- durch katalytische Hydrierung
durchzuführen

Unter komplexen Hydriden versteht man im Allgemeinen geladene Metallkomplexe, die wenigstens einen Hydrid-Liganden enthalten. Beispiele hierfür sind Lithiumaluminiumhydrid (LiAlH₄), LiAlH(O-tert-butyl)₃, LiAlH(O-methyl)₃, NaAlEt₂H₂ und dergleichen. Beispiele für nichtkomplexe Metall- und Halbmetallhydride sind Borane, wie BH₃, 9-BBN (9-Borabicyclo[3.3.1]nonan) und Disiamylboran, AlH₃, DIBAL-H (AlH(isobutyl)₂) und dergleichen.

Boran (BH₃) kann dabei gasförmig (als Diboran B₂H₆) oder in Lösung (z.B. als Etherat in der Form von BH₃-Komplexen, wie BH₃ - THF oder BH₃ - Me₂S oder BH₃ - Pyridin) eingesetzt werden. Werden Borhydride eingesetzt so diese erfindungsgemäß in-situ erzeugt. Beispielsweise kann eine in-situ Erzeugung von Borhydriden dadurch erfolgen, dass hydridische Borsalze, wie LiBH₄, NaBH₄ oder KBH₄ mit Lewis- oder Brønstedsäuren oder Halogenen, wie Iod, Brom oder Chlor, umgesetzt werden.

Beispiele für geeignete Lewissäuren sind Bor-, Aluminium oder Eisenhalogenidc.

Beispiele für geeignete Brønstedsäuren sind H₂SO₄, HCl oder Phosphorsäure. Ebenso kann die Erzeugung der Borhydride durch Reaktion von Borhalogeniden, wie BF₃, BCl₃ oder BBr₃, mit komplexen Hydriden, wie NaH oder LiAlH₄, erfolgen.

Die in diesem Fall zunächst gebildeten Aminborankomplexe können sowohl durch Zugabe einer geeigneten Säure als auch durch Zugabe einer Base in die freien Amine überführt werden. Geeignete Säuren können beispielsweise wässrige Salzsäure, Schwefelsäure und Phosphorsäure sein. Geeignete Basen sind zum Beispiel Natronlauge, Kalilauge und Ammoniakwasser. Die verwendeten Säuren oder Basen werden im Überschuß eingesetzt. Bevorzugt sind Mengen von 1,5 bis 2 Äquivalenten. Die Temperaturen können in einem weiten Bereich variiert werden. Bevorzugt sind Temperaturen zwischen 0 °C und 40 °C. Der pH kann während der Aminfreisetzung zwischen 0 und 14 variert werden. Im Falle der sauren Spaltung ist ein pH von 5 bis 2 bevorzugt. Im Falle der basischen Spaltung wird ein pH von 8 bis 12 bevorzugt.

Bei der Reduktion mit Si-Hydriden können beispielsweise Edelmetallkatalysatoren wie Rhodiumsalze oder Edelmetallkomplexe verwendet werden. Entsprechende Verfahrensweisen sind in Tetrahedron Letters, 39 (1998), Seiten 1017 bis 1020 beschrieben.

Wenn eine katalytische Hydrierung zur Reduktion der Verbindung der allgemeinen Formel (IV) angewendet wird, kann als Katalysator ein beliebiger Hydrierkatalysator verwendet werden. In Frage kommen beispielsweise Palladiumkatalysatoren, Platinkatalysatoren, Raney-NickelKatalysatoren, Lindlar-Katalysatoren und Rhodiumkatalysatoren. Neben diesen heterogenen Katalysatoren können jedoch auch Hydrierungen an homogenen Katalysatoren durchgeführt werden, beispielsweise an dem Wilkinson-Katalysator.

Die katalytische Hydrierung kann unter Überdruck in einem Autoklaven oder bei Normaldruck in einer Wasserstoffgasatmosphäre durchgeführt werden. Die Wasserstoffgasatmosphäre kann dabei zusätzlich auch inerte Gase, beispielsweise Argon oder Stickstoff, enthalten.

Im Allgemeinen ist es vorteilhaft, das erfindungsgemäße Verfahren in Gegenwart von Lösungsmitteln (Verdünnungsmitteln) durchzuführen. Lösungsmittel werden vorteilhaft in einer solchen Menge eingesetzt, dass das Reaktionsgemisch während des ganzen Verfahrens der Reduktion gut rührbar bleibt. Als Lösungsmittel zur Durchführung des erfindungsgemäßen Verfahrens kommen alle unter den Reaktionsbedingungen inerten organischen Lösungsmittel in Frage, wobei die Art des verwendeten Lösungsmittels von der Art der Reduktionsdurchführung, d.h. insbesondere von der Art des Reduktionsmittels, abhängt.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether, wie Ethylpropylether, Methyl-tert-butylether, Methyl-*n*-Butylether, Anisol, Phenetol, Cyclohexylmethylether, Dimethylether, Diethylether, Dimethylglycol, Diphenylether, Dipropylether, Diisopropylether, Di-*n*-butylether, Diisobutylether, Diisoamylether, Ethylenglycoldimethylether, Isopropylethylether, Methyl-tert-butylether, Tetrahydrofuran, Dioxan, Dichlordiethylether und Polyether des Ethylenoxids und/oder Propylenoxids; Amine wie Trimethyl-, Triethyl-, Tripropyl-, Tributylamin, *N*-Methylmorpholin, Pyridin, alkylierte Pyridine und Tetramethylendiamin; Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, Hexan, Heptan, Oktan, Nonan und technische Kohlenwasserstoffe welche durch Fluor- und Chloratome substituiert sein können, wie Methylenchlorid, Dichlormethan, Trichlormethan, Tetrachlorkohlenstoff, Fluorbenzol, Chlorbenzol oder Dichlorbenzol; beispielsweise sogenannte White Spirits mit Komponenten mit Siedepunkten im Bereich beispielsweise von 40°C bis 250°C, Cymol, Benzinfraktionen innerhalb eines Siedeinterwalles von 70°C bis 190°C, Cyclohexan, Methylcyclohexan, Petrolether, Ligroin, Octan, Benzol, Toluol, Chlorbenzol, Brombenzol, Xylol; Ester wie Methyl-, Ethyl-, Butyl-, Isobutylacetat, sowie Dimethyl-, Dibutyl- oder Ethylencarbonat.

Von den zuvor genannten Lösungsmitteln sind THF oder THF/Toluol-Gemische bevorzugt.

Die verwendeten Lösungsmittelmengen können in einem weiten Bereich variiert werden. Im Allgemeinen werden Lösungsmittelmengen im Bereich von der 1-fachen bis 50-fachen Lösungsmittelmenge, besonders bevorzugt von der 2-fachen bis 40-fachen Lösungsmittelmenge, insbesondere von der 2-fachen bis 30-fachen Lösungsmittelmenge, jeweils bezogen auf das eingesetzte substituierten 2,2-Difluoressigsäureamid der allgemeinen Formel (IV), verwendet.

In einer weiteren Ausgestaltung des erfindungsgemäßen Verfahrens kann auch ohne Lösungsmittel in der Schmelze des als Edukt eingesetzten 2,2-Difluoressigsäureamid-Derivats der allgemeinen Formel (IV) gearbeitet werden.

Die Reduktion erfolgt im Allgemeinen unter solchen Reaktionsbedingungen (Druck, Temperatur, Stöchiometrie etc.), unter denen die Carbonyl-Gruppe zur CH₂-Gruppe reduziert wird, gleichzeitig jedoch die übrigen im Molekül vorhandenen funktionellen Gruppen unverändert bleiben.

Bevorzugte Reaktionstemperaturen für die Reduktion mit komplexen Hydriden reichen von -20 °C bis 100 °C, wobei Temperaturen von 0 bis 30 °C bevorzugt sind. Die Reduktionen können bei Normaldruck oder auch unter erhöhten Drücken bis zu 200 bar erfolgen. Insbesondere bei höheren Reaktionstemperaturen kann es hilfreich sein auch bei erhöhten Drücken in einem Autoklaven zu arbeiten. Auch kann eine zusätzliche Druckerhöhung mittels eines zusätzlichen Inertgases, wie Stickstoff oder Argon, durchgeführt werden.

In einer bevorzugten Ausgestaltung des erfindungsgemäßen Verfahrens erfolgt die Hydrierung unter Normaldruck bei Raumtemperatur.

Das verwendete Reduktionsmittel wird in einem im Allgemeinen 1,1-bis 5-fachem molaren Überschuss, besonders bevorzugt 1,3- bis 4-fachem molaren Überschuss, insbesondere 1,5- bis 3-fachem molaren Überschuss, jeweils bezogen auf das eingesetzte 2,2-Difluoressigsäureamid-Derivat der allgemeinen Formel (IV), verwendet.

Die Reaktionsdauer der Hydrierung beträgt im Allgemeinen 30 Minuten bis 24 Stunden, wobei längere Reaktionszeiten sich nicht nachteilig auswirken.

Werden Katalysatoren eingesetzt, so kann deren Menge von 0,01 bis 10 Gewichtsprozent, bezogen auf das eingesetzte substituierte 2,2-Difluoressigsäureamid der allgemeinen Formel (IV), variiert werden.

Die Aufarbeitung und Reinigung kann über das freie Amin, über die Aminborankomplexe oder über Salze des Amins erfolgen. Das freie Amin wird bevorzugt durch Extraktionen und anschließende Destillation isoliert. Im Falle von Aminsalzen, beispielsweise von Salzen der organischen oder anorganischen Säuren, erfolgt eine Reinigung bevorzugt durch Kristallisation. Bevorzugte Salze sind zum Beispiel Hydrochloride oder Acetate. Wasserlösliche Salze können durch Extraktion der wässrigen Lösungen gereinigt werden. Das Amin kann dann schließlich durch Umsetzung mit organischen oder anorganischen Basen aus seinen Salzen freigesetzt werden. Bevorzugte Basen sind NaHCO₃, Na₂CO₃ oder NaOH.

Die vorliegende Erfindung betrifft darüber hinaus auch die Verwendung der Verbindungen der allgemeinen Formel (IV) zur Herstellung von Verbindungen der allgemeinen Formel (III), wie es in dem oben beschriebenen Verfahren offenbart ist.

Die für die erfindungsgemäße Umsetzung erforderlichen Verbindungen der allgemeinen Formel (IV) können dadurch erhalten werden, dass man Verbindungen der allgemeinen Formel (VI) 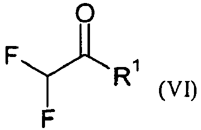 in welcher
- R¹: für Halogen, O-Alkyl, O-Alkylaryl oder Aryl steht
mit Aminen der allgemeinen Formel (VII) 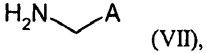 in welcher A die oben genannten Bedeutungen aufweist, zu einer Verbindung der allgemeinen Formel (IV) 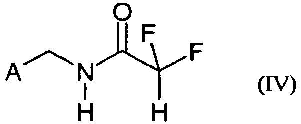 umsetzt.

Die für diese Umsetzung benötigten Verbindungen der allgemeinen Formel (VI) sind kommerziell erhältlich oder können nach literaturbekannten Verfahren hergestellt werden (Journal of Fluorine Chemistry, 31(4),363 - 79, 1986; EP 0 694 523 A; Jpn. Kokai Tokkyo Koho, 11343267).

In der Verbindung der allgemeinen Formel (VI) steht R¹ im Allgemeinen für Halogen, O-Alkyl oder O-Arylalkyl. Bevorzugt steht R¹ für Cl, F, O-C₁-C₆-Alkyl, besonders bevorzugt für OEt, OMe oder Cl.

Die für die Umsetzung zur Verbindung der allgemeinen Formel (IV) benötigten Amine der allgemeinen Formel (VII) sind kommerziell erhältlich oder können nach literaturbekannten Verfahren hergestellt werden (vgl. z.B. S. Patai "The Chemistry of Amino Group", Interscience Publishers, New York, 1968).

Die Umsetzung zur Herstellung der Verbindungen der allgemeinen Formel (IV) wird im Allgemeinen dergestalt durchgeführt, dass das Difluoressigsäure-Derivat der allgemeinen Formel (VI) vorgelegt und dann mit dem entsprechenden Amin der allgemeinen Formel (VII) umgesetzt wird. Die Umsetzung kann dabei bei einer Temperatur von im Allgemeinen 0 bis 150 °C, insbesondere 20 bis 130 °C, im Speziellen 20 bis 110 °C, durchgeführt werden. In einer besonders bevorzugten Ausführungsform wird diese zu den Verbindungen der allgemeinen Formel (IV) führende Umsetzung ohne den Zusatz eines Lösungsmittels durchgeführt, d.h. das Difluoressigsäure-Derivat der allgemeinen Formel (VI) wird vorgelegt und gleichzeitig als Lösemittel verwendet.

Die bei dieser Umsetzung entstehende Verbindung HR¹ - beispielsweise EtOH, wenn als Verbindung der allgemeinen Formel (VI) ein Ethylester verwendet wird - kann leicht, beispielsweise durch Destillation, von dem Rohprodukt abgetrennt werden.

Die Umsetzung zur Herstellung der Amide der allgemeinen Formel (IV) kann darüber hinaus auch in Gegenwart von Lösungsmitteln (Verdünnungsmitteln) durchgeführt werden. Die Lösungsmittel werden auch in diesem Verfahrensschritt vorzugsweise in einer solchen Menge eingesetzt, dass das Reaktionsgemisch während des ganzen Verfahrens der Reduktion gut rührbar bleibt. Als Lösungsmittel zur Durchführung des erfindungsgemäßen Verfahrens zur Herstellung der 2,2-Difluoramid-Derivate der allgemeinen Formel (VI) kommen alle unter den Reaktionsbedingungen inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether, wie Ethylpropylether, Methyl-tert-butylether, *n*-Butylether, Anisol, Phenetol, Cyclohexylmethylether, Dimethylether, Diethylether, Dimethylglycol, Diphenylether, Dipropylether, Diisopropylether, Di*n*-butylether, Diisobutylether, Diisoamylether, Ethylenglycoldimethylether, Isopropylethylether, Methyl-tert-butylether, Tetrahydrofuran, Dioxan, Dichlordiethylether und Polyether des Ethylenoxids und/oder Propylenoxids; Amine wie Trimethyl-, Triethyl-, Tripropyl-, Tributylamin, *N*-Methylmorpholin, Pyridin, alkylierte Pyridine und Tetramethylendiamin; Nitrokohlenwasserstoffe wie Nitromethan, Nitroethan, Nitropropan, Nitrobenzol, Chlornitrobenzol, o-Nitrotoluol; Nitrile wie Acetonitril, Methylnitril, Propionitril, Butyronitril, Isobutyronitril, Benzonitril, Phenylnitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, Hexan, Heptan, Oktan, Nonan und technische Kohlenwasserstoffe welche durch Fluor- und Chloratome substituiert sein können, wie Methylenchlorid, Dichlormethan, Trichlormethan, Tetrachlorkohlenstoff, Fluorbenzol, Chlorbenzol oder Dichlorbenzol; beispielsweise sogenannte White Spirits mit Komponenten mit Siedepunkten im Bereich beispielsweise von 40°C bis 250°C, Cymol, Benzinfraktionen innerhalb eines Siedeinterwalles von 70°C bis 190°C, Cyclohexan, Methylcyclohexan, Petrolether, Ligroin, Octan, Benzol, Toluol, Chlorbenzol, Brombenzol, Nitrobenzol, Xylol; Ester wie Methyl-, Ethyl-, Butyl-, Isobutylacetat, sowie Dimethyl-, Dibutyl-, Ethylencarbonat; Amide wie Hexamethylenphosphorsäuretriamid, Formamid, *N*-Methyl-formamid, *N,N*-Dimethyl-formamid, *N,N*-Dipropyl-formamid, *N,N*-Dibutyl-formamid, *N*-Methyl-pyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N*-Formyl-piperidin, *N,N'*-1,4-Diformyl-piperazin; Ketone wie Aceton, Acetophenon, Methylethylketon, Methylbutylketon. Von den vorgenannten Lösungsmitteln ist Toluol insbesondere bevorzugt.

Weiterer Gegenstand der vorliegenden Erfindung sind darüber hinaus die Verbindungen der allgemeinen Formel (IV), welche als Zwischenprodukte bei der Herstellung der Zielverbindungen der allgemeinen Formel (III) verwendet werden:

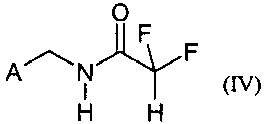

In diesen Intermediaten weist der Substituent A im Allgemeinen die folgende Bedeutung auf:
- Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder
- Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder 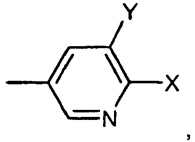
in welchem
- X: für Halogen, Alkyl oder Halogenalkyl steht und
- Y: für Halogen, Alkyl, Halogenalkyl, Halogenalkoxy, Azido oder Cyan steht.

Bevorzugte, besonders bevorzugte und ganz besonders bevorzugte Substituenten bzw. Bereiche des in den oben erwähnten allgemeinen Formeln (III) und (IV) aufgeführten Restes A werden im Folgenden erläutert.
- A: wird bevorzugt ausgewählt aus der Gruppe, bestehend aus 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-1,3-thiazol-5-yl, 2-Chlor-pyrimidin-5-yl, 2-Trifluormethyl-pyrimidin-5-yl, 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3-yl, 5-Methyl-6-iod-pyrid-3-yl, 5-Difluormethyl-6-fluor-pyrid-3-yl, 5-Difluormethyl-6-chlor-pyrid-3-yl, 5-Difluormethyl-6-brom-pyrid-3-yl und 5-Difluormethyl-6-iod-pyrid-3-yl.
- A: wird besonders bevorzugt ausgewählt aus der Gruppe, bestehend aus 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl, 2-Chlor-pyrimidin-5-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl und 5-Difluormethyl-6-chlor-pyrid-3-yl.
- A: wird ganz besonders bevorzugt ausgewählt aus der Gruppe, bestehend aus 6-Chlor-pyrid-3-yl, 6-Brom-pyrid-3-yl, 6-Chlor-1,4-pyridazin-3-yl, 2-Chlor-1,3-thiazol-5-yl, 5-Fluor-6-chlor-pyrid-3-yl und 5-Fluor-6-brom-pyrid-3-yl.

Die Verbindungen der allgemeinen Formel (IV) können als Edukt zur Herstellung der 2,2-Difluoramid-Derivate der allgemeinen Formel (IV) verwendet werden.

Ausgehend von den Verbindungen der allgemeinen Formel (III), welche durch das erfindungsgemäße Verfahren erhalten werden, können insektizid wirksame Enaminocarbonylverbindungen, welche 2,2-Difluorethylaminobausteine umfassen und beispielsweise in den internationalen Patentanmeldungen WO 2007/115644 und WO 2007/115646 beschrieben sind, hergestellt werden.

Zu diesem Zweck werden die Verbindungen der allgemeinen Formel (III) 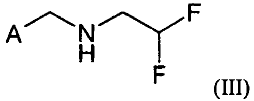 an dem sekundären Aminstickstoff beispielsweise durch Umsetzung mit Tetronsäure oder Derivaten davon alkenyliert. Eine entsprechende Umsetzung ist in der Schema I der WO 2007/115644 näher beschrieben und führt unmittelbar zu den insektizid wirksamen Enaminocarbonylverbindungen.

Die vorliegende Erfindung wird anhand der nachfolgenden Beispiele näher erläutert, wobei die Beispiele nicht in die Erfindung einschränkender Weise zu interpretieren sind.

### Herstellungsbeispiele:

### Beispiel 1

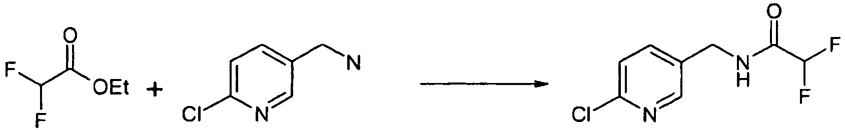

124 g (1 mol) 1,1-Di-Fluoressigsäureethylester werden vorgelegt und mit 142,6 g 2-Chlor-5-aminomethylpyridin versetzt. Das Reaktionsgemisch wird auf 100 °C erwärmt und eine Stunde unter Rückfluss gekocht. Nach Abstellen der Kühlung wird das entstandene Ethanol überdestilliert. Gegen Ende der Umsetzung wird zur vollständigen Entfernung des Ethanols bis zu einer Sumpftemperatur von 115 °C erwärmt oder ein schwaches Vakuum angelegt. Im Reaktionskolben verbleiben 221 g 99 %iges Amid (99% d. Th.), welches bei etwa 90 °C erstarrt.

NMR (d-DMSO): NH (br s,9,4 ppm); 1H(s, 8,35 ppm); 1H (d,7,75 ppm); 1H (d,7,5 ppm); 1 H (t, 6,1-6,4 ppm); 2 H (s, 4,4 ppm).

### Beispiel 2

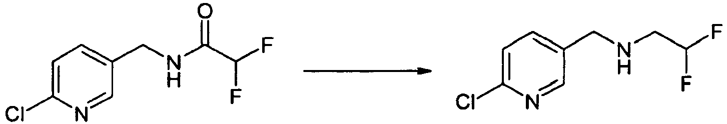

22,6 g (0,1 mol) 1,1-Di-Fluoressigsäure-(2-chlor-5-aminomethyl-pyridin)amid werden in 200 ml THF nach Zugabe von 1 g RhCl₃ · 3 H2O vorgelegt. Dann werden 21 g Phenylsilan zugegeben und bis zum vollständigen Umsatz nachgerührt. Danach wird mit 1 N Salzsäure versetzt und mit Diethylether extrahiert. Die wässrige Phase wird mit 15 %iger Natronlauge alkalisch gestellt und mit Diethylether extrahiert. Nach Trocknen über Na₂SO₄ wird das Lösungsmittel unter vermindertem Druck abdestilliert. Es verbleiben 20 g 98 % iges Amin (95 % d. Th.).

NMR(d-DMSO): 1H(s, 8,35 ppm); 1H (d,7,8 ppm); 1H (d,7,46 ppm); 1 H (td, 6,02 ppm); 2 H (s, 3,8 ppm); 2 H (td, 2,9 ppm)

### Beispiel 3

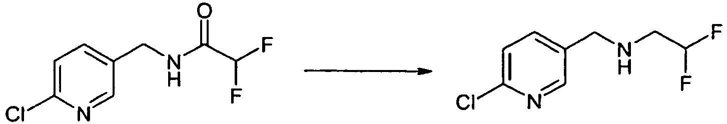

20 g (0,091 mol) 1,1-Di-Fluoressigsäure-(2-chlor-5-aminomethyl-pyridin)amid werden in 200 ml THF vorgelegt. Nach der Zugabe von 6,86 g NaBH₄ wird auf 0 bis 5 °C abgekühlt und 34,3 g BF₃ · Etherat zugetropft. Es wird über Nacht bei 0 bis 5 °C nachgerüht. Zur Aufarbeitung wird Ethanol und anschließend verdünnte Salzsäure zugetropft und einige Stunden nachgerührt. Nach Abdestillieren der Hauptmenge THF unter vermindertem Druck wird die Wasserphase mit Ether extrahiert. Die wässrige Phase wird mit 15 %iger Natronlauge auf pH 12 gestellt und 2 mal mit Diethylether extrahiert. Nach Trocknen über Na₂SO₄ wird das Lösungsmittel unter vermindertem Druck abdestilliert. Es verbleiben 17,75 g 95 %iges Amin (90 % d. Th).
NMR-Daten: siehe Beispiel 2

### Beispiel 4

20 g (0,091 mol) 1,1-Di-Fluoressigsäure-(2-chlor-5-aminomethyl-pyridin)amid werden in 200 ml THF vorgelegt. Nach der Zugabe von 6,86 g NaBH₄ wird auf 0 bis 5 °C abgekühlt und 34,3 g BF₃ · Etherat zugetropft. Es wird über Nacht bei 0 bis 5 °C nachgerüht. Zur Aufarbeitung wird Ethanol und anschließend verdünnte Natronlauge zugetropft und einige Stunden bei 40 °C nachgerührt. Der pH der Lösung beträgt 12. Nach Abdestillieren der Hauptmenge THF unter vermindertem Druck wird die Wasserphase 2 mal mit Diethylether extrahiert. Nach Trocknen über Na₂SO₄ wird das Lösungsmittel unter vermindertem Druck abdestilliert. Es verbleiben 17 g 95 %iges Amin (86 % d. Th).

## Patentansprüche

1. Verfahren zur Herstellung von 2,2-Difluorethylamin-Derivaten der Formel (III) 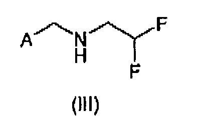 umfassend die folgenden Schritte (i) und (ii);
Schritt (i): Herstellung eines 2,2-Difluoressigsäureamid-Derivats der Formel (IV) 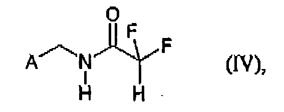 durch die Umsetzung einer Verbindung der Formel (VI) 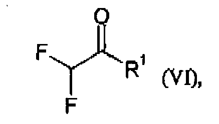 in welcher R¹ für Halogen, O- C₁-C₁₂-Alkyl oder O-Aryl-C₁-C₁₂-alkyl steht, mit einem Amin der Formel (VII) 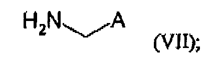 und
Schritt (ii): Reduktion des 2,2-Difluoressigsäureamid-Derivats der Formel (IV) unter Verwendung eines Reduktionsmittels, das ausgewählt ist aus der Gruppe, bestehend aus komplexen Hydriden, in-situ erzeugten Borhydriden, und Hydriden von siliziumhaltigen Verbindungen der Formel H-Si-R₃, in der R für H, gegebenenfalls substituiertes C₁-C₁₂-Alkyl, gegebenenfalls substituiertes Aryl oder Chlor steht, oder durch katalytische Hydrierung,
wobei in den Formeln (III), (IV) und (VII) der Rest A
für Pyrid-2-yl oder Pyrid-4-yl oder für Pyrid-3-yl, welches gegebenenfalls in 6-Position substituiert ist durch Fluor, Chlor, Brom, Methyl, Trifluormethyl oder Trifluormethoxy oder für Ryridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder
für Pyrimidinyl, Pyrazolyl, Thiophenyl, Oxazolyl, Isoxezolyl, 1,2,4-Oxadiazolyl, Isothiazolyl, 1,2,4-Triazolyl oder 1,2,5-Thiadiazolyl, welcher gegebenenfalls durch Fluor, Chlor, Brom, Cyano,
Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist steht, oder
für einen Rest der folgenden Formel steht, 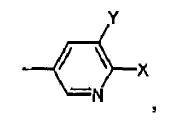 in welcher
X für Halogen, C₁-C₁₂-Alkyl oder C₁-C₁₂-Halogenalkyl steht, und
Y für Halogen, C₁-C₁₂-Alkyl, C₁-C₁₂-Halogenalkyl, C₁-C₁₂-Halogenalkoxy, Azido oder Cyan steht.

2. Verfahren nach Anspruch 1, worin im Schritt (ii) die komplexen Hydride ausgewählt sind unter Lithiumaluminiumhydrid (LiAlH₄), LiAlH(O-tert-butyl)₃, LiAlH(O-methyl), und NaAlEt₂H₂,

3. Verfahren nach Anspruch 1, worin im Schritt (ii) die in-situ erzeugten Borhydride durch Zugabe von hydridische Borsalzen, die ausgewählt sind unter LiBH₄, NaBH₄ oder KBH₄, mit Lewis- oder Brønstedsäuren oder Halogenen erzeugt werden, oder die durch Zugabe von Borhalogeniden, die ausgewählt sind unter BF₃, BCl₃ und BBr₃, und komplexen Hydriden, die ausgewählt sind unter NaH und LiAlH₄, erzeugt werden.

4. Verfahren nach Anspruch 3, worin in Schritt (ii) die Lewissäuren ausgewählt sind unter Bor-, Aluminium- oder Eisenhalogeniden und die Brønstedsäuren ausgewählt sind unter H₂SO₄, HCl oder Phosphorsäure.

5. Verfahren nach Anspruch 3 oder 4, worin im Schritt (ii) das Reduktionsmittel ein in-situ erzeugtes Borhydrid ist und, das einen weiteren Schritt (iii) umfasst, nämlich
Schritt (iii): Überführung der zunächst gebildeten Aminborankomplexe durch Zugabe einer Säure oder Zugabe einer Base in die freien Amine, wobei die Säure ausgewählt ist unter wässriger Salzsäure, Schwefelsäure und Phosphorsäure, und wbei die Base ausgewählt ist unter Natronlauge, Kalilauge und Ammoniakwasser, wobei die Säure oder Base im Überschuss vorliegen.

6. Verfahren nach Anspruch 5, wobei Schritt (iii) bei einer Temperatur zwischen 0 °C und 40 °C durchgeführt wird.

7. Verfahren nach Anspruch 5 oder 6, wobei in Schritt (iii) der pH während der Aminfreisetzung im Falle der sauren Spaltung im Bereich von 5 bis 2 liegt und im Falle der basischen Spaltung im Bereich von 8 bis 12 liegt.

8. Verbindungen der Formel (IV) 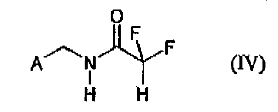 in der Rest A
für Pyrid-2-yl oder Pyrid-4-yl oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder
für Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist steht, oder
für einen Rest der folgenden Formel steht, 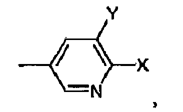 in welcher
X für Halogen, C₁-C₁₂-Alkyl oder C₁-C₁₂-Halogenalkyl steht, und
Y für Halogen, C₁-C₁₂-Alkyl, C₁-C₁₂-Halogenalkyl, C₁-C₁₂-Halogenalkoxy, Azido oder Cyan steht,

## Claims

1. Process for preparing 2,2-difluoroethylamine derivatives of the formula (III) 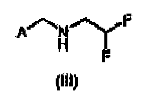 comprising the following steps (i) and (ii):
step (i): preparation of a 2,2-difluoroacetamide derivative of the formula (IV) 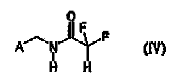 by the reaction of a compound of the formula (VI) 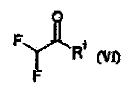 in which R¹ is halogen, O-C₁-C₁₂-alkyl or O-aryl-C₁-C₁₂-alkyl
with an amine of the formula (VII) 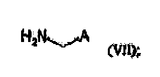 and
step (ii): reduction of the 2,2-difluoroacetamide derivative of the formula (IV) using a reducing agent selected from the group consisting of complex hydrides, borohydrides produced in situ, and hydrides of silicon compounds of the formula H-Si-R₃ in which R is H, optionally substituted C₁-C₁₂-alkyl, optionally substituted aryl or chlorine, or by catalytic hydrogenation,
where the A radical in the formulae (III), (IV) and (VII) is
pyrid-2-yl or pyrid-4-yl, or pyrid-3-yl which is optionally 6-substituted by fluorine, chlorine, bromine, methyl, trifluoromethyl or trifluoromethoxy, or pyridazin-3-yl which is optionally 6-substituted by chlorine or methyl, or pyrazin-3-yl, or 2-chloropyrazin-5-yl, or 1,3-thiazol-5-yl which is optionally 2-substituted by chlorine or methyl, or
pyrimidinyl, pyrazolyl, thiophenyl, oxazolyl, isoxazolyl, 1,2,4-oxadiazolyl, isothiazolyl, 1,2,4-triazolyl or 1,2,5-thiadiazolyl, which is optionally substituted by fluorine, chlorine, bromine, cyano, nitro, C₁-C₄-alkyl (which is optionally substituted by fluorine and/or chlorine), C₁-C₃-alkylthio (which is optionally substituted by fluorine and/or chlorine), or C₁-C₃-alkylsulphonyl (which is optionally substituted by fluorine and/or chlorine), or
is a radical of the following formula 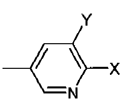 in which
X is halogen, C₁-C₁₂-alkyl or C₁-C₁₂-haloalkyl and
Y is halogen, C₁-C₁₂-alkyl, C₁-C₁₂-haloalkyl, C₁-C₁₂-haloalkoxy, azido or cyano.

2. Process according to Claim 1, in which the complex hydrides in step (ii) are selected from lithium aluminium hydride (LiAlH₄), LiAlH(O-tert-butyl)₃, LiAlH(O-methyl)₃ and NaAlEt₂H₂.

3. Process according to Claim 1, in which the borohydrides produced in situ in step (ii) are produced by addition of hydridic boron salts selected from LiBH₄, NaBH₄ and KBH₄ with Lewis or Brønsted acids or halogens, or which are produced by addition of boron halides selected from BF₃, BCl₃ and BBr₃, and complex hydrides selected from NaH and LiAlH₄.

4. Process according to Claim 3, in which the Lewis acids in step (ii) are selected from boron halides, aluminium halides and iron halides, and the Brønsted acids are selected from H₂SO₄, HCl and phosphoric acid.

5. Process according to Claim 3 or 4, in which the reducing agent in step (ii) is a borohydride produced in situ, and which comprises a further step (iii), namely:
step (iii): conversion of the amine-borane complexes initially, formed by addition of an acid or addition of a base to the free amines, the acid being selected from aqueous hydrochloric acid, sulphuric acid and phosphoric acid, and the base being selected from aqueous sodium hydroxide solution, aqueous potassium hydroxide solution and aqueous ammonia, the acid or base being present in excess.

6. Process according to Claim 5, wherein step (iii) is performed at a temperature between 0°C and 40°C.

7. Process according to Claim 5 or 6, wherein the pH during the amine release in step (iii) is in the range from 5 to 2 in the case of acidic splitting and in the range from 8 to 12 in the case of basic splitting.

8. Compounds of the formula (IV) 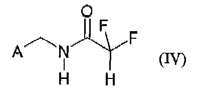 in which radical A is
pyrid-2-yl or pyrid-4-yl, or pyrid-3-yl which is optionally 6-substituted by fluorine, chlorine, bromine, methyl, trifluoromethyl or trifluoromethoxy, or pyridazin-3-yl which is optionally 6-substituted by chlorine or methyl, or pyrazin-3-yl, or 2-chloropyrazin-5-yl, or 1,3-thiazol-5-yl which is optionally 2-substituted by chlorine or methyl, or
pyrimidinyl, pyrazolyl, thiophenyl, oxazolyl, isoxazolyl, 1,2,4-oxadiazolyl, isothiazolyl, 1,2,4-triazolyl or 1,2,5-thiadiazolyl, which is optionally substituted by fluorine, chlorine, bromine, cyano, nitro, C₁-C₄-alkyl (which is optionally substituted by fluorine and/or chlorine), C₁-C₃-alkylthio (which is optionally substituted by fluorine and/or chlorine), or C₁-C₃-alkylsulphonyl (which is optionally substituted by fluorine and/or chlorine), or
is a radical of the following formula 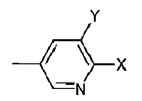 in which
X is halogen, C₁-C₁₂-alkyl or C₁-C₁₂-haloalkyl and
Y is halogen, C₁-C₁₂-alkyl, C₁-C₁₂-haloalkyl, C₁-C₁₂-haloalkoxy, azido or cyano.

## Revendications

1. Procédé pour la préparation de dérivés de 2,2-difluoroéthylamine de formule (III) 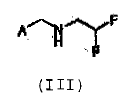 comprenant les étapes (i) et (ii) suivantes :
étape (i) : préparation d'un dérivé de 2,2-difluoroacétamide de formule (IV) 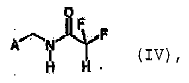 par la mise en réaction d'un composé de formule (VI), 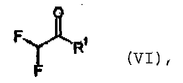 dans laquelle R¹ représente un atome d'halogène, un groupe O-alkyle(C₁-C₁₂) ou O-aryl-alkyle(C₁-C₁₂),
avec une amine de formule (VII) 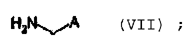 et
étape (ii) : réduction du dérivé de 2,2-difluoro-acétamide de formule (IV) à l'aide d'un réducteur qui est choisi dans le groupe constitué par des hydrures complexes, des borohydrures engendrés in situ, et des hydrures de composés siliciés de formule H-Si-R₃, dans laquelle R représente H, un groupe alkyle en C₁-C₁₂ éventuellement substitué, un groupe aryle éventuellement substitué ou un atome de chlore, ou par hydrogénation catalytique,
le radical A dans les formules (III), (IV) et (VII)
représentant un groupe pyridyl-2-yle ou pyrid-4-yle ou représentant un groupe pyrid-3-yle qui est éventuellement substitué en position 6 par fluoro, chloro, bromo, méthyle, trifluorométhyle ou trifluorométhoxy, ou représentant un groupe pyridazin-3-yle qui est éventuellement substitué en position 6 par chloro ou méthyle ou représentant un groupe pyrazin-3-yle ou représentant un groupe 2-chloro-pyrazin-5-yle ou représentant un groupe 1,3-thiazol-5-yle qui est éventuellement substitué en position 2 par chloro ou méthyle, ou
représentant un groupe pyrimidinyle, pyrazolyle, thiophényle, oxazolyle, isoxazolyle, 1,2,4-oxadiazolyle, isothiazolyle, 1,2,4-triazolyle ou 1,2,5-thiadiazolyle, qui est éventuellement substitué par fluoro, chloro, bromo, cyano, nitro, alkyle en C₁-C₄ (qui est éventuellement substitué par fluoro et/ou chloro), alkyl(C₁-C₃)thio (qui est éventuellement substitué par fluoro et/ou chloro), ou alkyl(C₁-C₃)-sulfonyle (qui est éventuellement substitué par fluoro et/ou chloro), ou
représentant un radical de formule suivante, 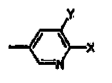 dans laquelle
X représente un atome d'halogène, un groupe alkyle en C₁-C₁₂ ou halogénoalkyle(C₁-C₁₂), et
Y représente un atome d'halogène, un groupe alkyle en C₁-C₁₂, halogénoalkyle(C₁-C₁₂), halogéno-alcoxy(C₁-C₁₂), azido ou cyano.

2. Procédé selon la revendication 1, dans lequel les hydrures complexes dans l'étape (ii) sont choisis parmi l'hydrure de lithium et d'aluminium (LiAlH₄), LiAlH(O-tert-butyle)₃, LiAlH(O-méthyle)₃, et NaAlEt₂H₂.

3. Procédé selon la revendication 1, dans lequel dans l'étape (ii) les borohydrures engendrés in situ sont produits par addition de sels de bore hydrurés, qui sont choisis parmi LiBH₄, NaBH₄ ou KBH₄, avec des acides de Brønsted ou des halogènes, ou qui sont produits par addition d'halogénures de bore qui sont choisis parmi BF₃, BCl₃ et BBr₃, et des hydrures complexes qui sont choisis parmi NaH et Li-AlH₄.

4. Procédé selon la revendication 3, dans lequel les acides de Lewis dans l'étape (ii) sont choisis parmi les halogénures de bore, d'aluminium ou de fer et les acides de Brønsted sont choisis parmi H₂SO₄, HCl ou l'acide phosphorique.

5. Procédé selon la revendication 3 ou 4, dans lequel le réducteur dans l'étape (ii) est un hydrure de bore engendré in situ, et qui comprend une autre étape (iii), à savoir
étape (iii) : conversion des complexes d'aminoborane formés en premier lieu, par addition d'un acide ou addition d'une base, en les amines libres, l'acide étant choisi parmi les acides chlorhydrique, sulfurique et phosphorique aqueux, et la base étant choisie parmi une solution d'hydroxyde de sodium, une solution d'hydroxyde de potassium et l'ammoniaque, l'acide ou la base se trouvant en excès.

6. Procédé selon la revendication 5, dans lequel on effectue l'étape (iii) à une température comprise entre 0 °C et 40 °C.

7. Procédé selon la revendication 5 ou 6, dans lequel dans l'étape (iii) le pH pendant la libération d'amines se situe, dans le cas de la coupure acide, dans l'intervalle de 5 à 2 et, dans le cas de la coupure basique, dans l'intervalle de 8 à 12.

8. Composés de formule (IV) 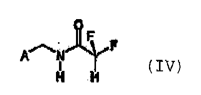 dans laquelle le radical A
représente un groupe pyridyl-2-yle ou pyrid-4-yle ou représente un groupe pyrid-3-yle qui est éventuellement substitué en position 6 par fluoro, chloro, bromo, méthyle, trifluorométhyle ou trifluorométhoxy, ou représente un groupe pyridazin-3-yle qui est éventuellement substitué en position 6 par chloro ou méthyle ou représente un groupe pyrazin-3-yle ou représente un groupe 2-chloropyrazin-5-yle ou représente un groupe 1,3-thiazol-5-yle qui est éventuellement substitué en position 2 par chloro ou méthyle, ou
représente un groupe pyrimidinyle, pyrazolyle, thiophényle, oxazolyle, isoxazolyle, 1,2,4-oxadiazolyle, isothiazolyle, 1,2,4-triazolyle ou 1,2,5-thiadiazolyle, qui est éventuellement substitué par fluoro, chloro, bromo, cyano, nitro, alkyle en C₁-C₄ (qui est éventuellement substitué par fluoro et/ou chloro), alkyl(C₁-C₃)thio (qui est éventuellement substitué par fluoro et/ou chloro), ou alkyl(C₁-C₃)-sulfonyle (qui est éventuellement substitué par fluoro et/ou chloro), ou représente un radical de formule suivante, 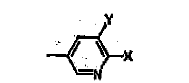 dans laquelle
X représente un atome d'halogène, un groupe alkyle en C₁-C₁₂ ou halogénoalkyle(C₁-C₁₂), et
Y représente un atome d'halogène, un groupe alkyle en C₁-C₁₂, halogénoalkyle(C₁-C₁₂), halogéno-alcoxy(C₁-C₁₂), azido ou cyano.
